# EUROPEAN PATENT SPECIFICATION

(11) **EP 1 617 896 B1**
(45) Date of publication and mention of the grant of the patent: **02.09.2009**
(21) Application number: 04760121.6
(22) Date of filing: 22.04.2004
(51) Int. Cl.: A61N 1/18, A61N 1/39

(54) **DEFIBRILLATOR/MONITOR SYSTEM HAVING A POD WITH LEADS CAPABLE OF WIRELESSLY COMMUNICATING**
HERZSCHRITTMACHER/ÜBERWACHUNGSGERÄT ENTHALTEND EINEN BEHÄLTER MIT DRÄHTEN ZUR DRAHTLOSEN KOMMUNIKATION
SYSTEME DE DEFIBRILLATION/SURVEILLANCE COMPORTANT UN ENSEMBLE FONCTIONNEL POURVU D'ELECTRODES, POUVANT EFFECTUER DES COMMUNICATIONS SANS FIL

(30) Priority: 22.04.2003 US 464860 P; 17.12.2003 US 530151 P
(43) Date of publication of application: 25.01.2006
(73) Proprietor: Medtronic Physio-Control Corp., Redmond, WA 98073-9706 (US)
(72) Inventor: PEARCE, Christopher, Redmond, WA 98073-9706 (US); NEUMILLER, James, Redmond, WA 98073-9706 (US); DAYNES, John, C., Redmond, WA 98073-9706 (US); HILL, Doug, Redmond, WA 98073-9706 (US); KAVOUNAS, Gregory, T., 11410 N.E. 124th Street, Kirkland, WA 98034 (US); MCGRATH, Thomas, J., Redmond, WA 98073-9706 (US); MERRY, Randy, Redmond, WA 98073-9706 (US); PETERSON, Ken, Redmond, WA 98073-9706 (US); WUNG, Peter, Redmond, WA 98073-9706 (US)
(74) Representative: Hughes, Andrea Michelle
(86) International application number: PCT/US2004/012421
(87) International publication number: WO 2004/093979

(56) References cited:
- WO-A-01/66182
- US-A- 3 865 101
- US-A1- 2002 133 201
- US-A1- 2003 028 219

## Description

### TECHNICAL FIELD

The field relates to medical devices, and in particular, to defibrillation/monitor systems having a detachable pod with leads.

### BACKGROUND

Each day thousands of Americans are victims of cardiac emergencies. Cardiac emergencies typically strike without warning, oftentimes striking people with no history of heart disease. The most common cardiac emergency is sudden cardiac arrest ("SCA"). It is estimated more than 1000 people per day are victims of SCA in the United States alone.

SCA occurs when the heart stops pumping blood. Usually SCA is due to abnormal electrical activity in the heart, resulting in an abnormal rhythm (arrhythmia). One such abnormal rhythm, ventricular fibrillation (VF), is caused by abnormal and very fast electrical activity in the heart. During VF the heart cannot pump blood effectively. Because blood may no longer be pumping effectively during VF, the chances of surviving decreases with time after the onset of the emergency. Brain damage can occur after the brain is deprived of oxygen for four to six minutes.

Applying an electric shock to the patient's heart through the use of a defibrillator treats VF. The shock clears the heart of the abnormal electrical activity (in a process called "defibrillation") by depolarizing a critical mass of myocardial cells to allow spontaneous organized myocardial depolarization to resume.

Cardiac arrest is a life-threatening medical condition that may be treated with external defibrillation. External defibrillation includes applying electrodes to the patient's chest and delivering an electric shock to the patient to depolarize the patient's heart and restore normal sinus rhythm. The chance a patient's heart can be successfully defibrillated increases significantly if a defibrillation pulse is applied quickly.

In a scenario where a paramedic is responding to an emergency call with a nonspecific patient condition, for example, there has been a car accident. The paramedic will typically carry his or her own defibrillator/monitor, a gurney, and drug box, and other supplies considered essential. If, perhaps, the car has driven off an embankment, the paramedic will have a long distance to run with all this equipment. This slows the response time to a call where someone may be bleeding to death. Smaller lighter equipment is always demanded by paramedics to save them time and effort, and allow them to get to the scene earlier. For just this reason, some paramedics will opt to carry only an AED (Automatic External Defibrillator) to the scene, and move the patient into the ambulance as quickly as possible, where other, more advanced monitoring equipment is available. In some countries, this approach has been incorporated into standard operating protocols, where the ambulance carries both ALS (advanced life support) equipment (which typically would include a multi-parameter monitor and defibrillator) and an AED. This approach, while effectively giving the user the choice of equipment to carry, forces the paramedic to learn two different defibrillators. The approach also forces the paramedics to possibly transfer the patient from one machine to the other once in the ambulance. It also adds costs to the ambulance service and potentially causes lost data between the two defibrillators for critical minutes, which may negatively impact the ability of EP Lab (Electro-Physiology Lab) doctors to determine the original cardiac condition.

Previous attempts to address the issue of product weight have done so by creating a manual defibrillator that separates from a patient monitor, or an AED, which separates from a single-channel patient monitor, or a manual defibrillator/pacemaker that separates from a 12-lead ECG monitor. These products suffer from limitations by the present standards, such as: limited capture of patient data, limited ability to monitor all necessary patient vital signs, and possible unreliability due to the nature of the electrical contacts between the two devices (e.g., dirt, mud, and damage to the case which could affect alignment of electrical contacts, thus preventing full functionality of the devices when mated).

In a scenario where a patient on a gurney is being transported through narrow doorways and down stairwells to an ambulance, or the situation where a patient is in an ambulance moving on a road at high speed with patient cables and IV (intravenous) lines running between the patient and other equipment within the ambulance, problems exist. If the monitoring/therapeutic device is large or the route to the ambulance is particularly difficult, the paramedic might elect to carry the device separately from the gurney to prevent the device falling off the gurney or onto the patient. However, the paramedic is now restricted in his or her ability to detach the device from the gurney due to the number and length of patient cables between the device and the patient. Similar restrictions occur once the patient is loaded into a patient transport vehicle or when the patient is transferred from the ambulance to the emergency department. The number of cables and their similarity in color or dissimilarity in length can all contribute to delays in treating or transferring the patient and can restrict the paramedics mobility when treating the patient in a confined space. Additionally, delays may be created with cables having become tangled, or even cut, from their previous uses.

The prior art has tried to solve this problem by providing a wireless module that transmits data to a patient monitor, such as the MobiMed offered for Sale by Ortivus. However, this device does not include a defibrillator and does not have the capability to provide any therapeutic functions such as pacing, defibrillation or synchronous cardioversion without attaching another monitor/defibrillator to the patient, which further increases the complexity and ambulance provider cost. Additionally, the Ortivus patient module does not offer replaceable batteries so functionality is severely limited if a reliable source of battery charging is not available, or if the transport time is excessively long. Additionally, the Ortivus device does not offer a display to allow visual monitoring of the waveforms or vital signs if the other module is out of range or obscured.

Another problem arises when hospital personnel want to charge the batteries of the defibrillator/monitor, but don't want to have to place the unit in a docking station in order to charge the batteries. There also arises the issue of patient confidentiality, such as recently raised by the Federal HIPAA (Health Insurance Portability and Accountability Act) regulations, when identical looking patient monitors are accidentally swapped by users.

Another problem may occur in a situation where two or more sets of paired wireless devices are used in the same general area. This type of problem could occur in a number of different (medical or non-medical) applications. For example, medical device A is comprised of two parts, a patient data acquisition module (AA) and a display module (AD). The two parts communicate with each other via one of many wireless methods. Medical device B is comprised of two similar parts patient data acquisition module (BA) and display module (BD). In the event of a mass casualty incident, where medical personnel are attending to more than one patient, two or more patients may be laying close to each other. Suppose patient X is being attended to by the user of device A, and a different user who is using device B is attending to patient Y. Patient X's vital signs are being acquired by acquisition module AA and transmitted to display module AD. Patient Y's vital signs are being acquired by acquisition module BA and transmitted to display module BD. A problem would arise when, in the state of confusion typically existing in a mass casualty incident, the two display modules become switched. In this case, the user of display module AD would be viewing the vital signs transmitted from Patient X while attending to Patient Y. This could result in inappropriate administration of drugs or other therapy with potentially serious consequences. The acquisition modules would still be paired to the appropriate display modules, and would still be functioning properly, but the user would be viewing the wrong patient's vital signs.

Other problems with wireless communications include the fact wireless communications methods cannot be visually assessed by the user prior to failure, such as a broken or damaged cable can. Wireless communications may not be permitted in certain areas, such as an aircraft environment, in military use, or elsewhere. Some wireless communications means have delays between sending a message and getting a response which are too long for therapeutic and other needs. There is a risk of the user not being able to find a cable when, for instance, a critical therapy has to be administered where the wireless link cannot support it.
WO 01/66182 and US 2003/0028219 disclose modular defibrillators.

### SUMMARY

A modular external defibrillator system in embodiments of the invention may include one or more of the following features: (a) a base containing a defibrillator module, (b) a pod having a patient parameter module with patient lead cables attachable to a patient to collect at least one patient vital sign, the pod operable at a distance from the base, and (c) a communications link between the pod and the base to carry the at least one vital sign from the pod to the base, the defibrillator module delivering a defibrillation shock to the patient based on the at least one vital sign.

A modular external defibrillator system in embodiments of the invention may include one or more of the following features: (a) a base containing a defibrillator module adapted to deliver a defibrillation shock to a patient, (b) a pod having a patient parameter module with patient lead cables attachable to the patient to collect patient vital signs, the pod operable at a distance from the base, (c) a communications link between the pod and the base to carry the patient vital signs from the pod to the base, the base having a monitor area to visually display the patient vital signs, (d) the communications link is a direct electrical connection between the pod and the base, (e) the communications link is a wireless communications link, and (f) a direct electrical connection between the pod and the base serves as an alternate communications link to the wireless communications link, (f) the communications link is a cable tethered to and housed within the base, (g) the tethered cable is retractable into the base when not in use, (h) a first end of the cable is coupled to a base interface connector located within a connector cavity of the base and a second end of the cable is connected to the base, (i) the first end of the tethered cable can be removed from the cavity to provide the direct electrical connection between the base and pod when the pod is not attached to the base, (j) the patient vital signs monitored by the pod include one or more of multi-lead ECG data, non-invasive blood pressure data, pulse oximeter data, capnography data and respiratory data, invasive blood pressure readings, and patient temperature data, (k) the base monitor area visually displays one or more of multi-lead ECG data, non-invasive blood pressure data, pulse oximeter data, capnography data, invasive blood pressure readings, and patient temperature data, (l) the pod includes a monitor area to visually display patient data, (m) the pod monitor area visually displays one or more of multi-lead ECG data, non-invasive blood pressure data, pulse oximeter data, capnography data, invasive blood pressure readings, and patient temperature data, (n) the defibrillator module synchronizes defibrillation shocks to the patient's intrinsic rhythm based on the patient vital signs, and (o) the base includes a data interpretation module which analyzes the patient vital signs to form interpretive statements on the patient's cardiac or respiratory condition.

An external cardiac therapy system in embodiments of the invention may include one or more of the following features: (a) a pod having a patient parameter module with patient leads attachable to a patient to collect patient data, (b) a base containing a cardiac therapy module adapted to deliver an electrical cardiac therapy to the patient, the base having a latching assembly to mount the pod in a releasable manner, the pod operable at a distance from the base, (c) a communications link between the pod and the base to transfer the patient data from the pod to the base, the base having a display area to visually display the patient data, (d) the latching assembly has a recess to receive the pod, (e) the recess can releasably hold one or more pods, and (f) the recess releasably mounts two of the pods.

An external cardiac therapy system in embodiments of the invention may include one or more of the following features: (a) a pod having a patient parameter module with patient leads attachable to a patient to collect patient data, (b) a base containing a cardiac therapy module adapted to deliver an electrical cardiac therapy to the patient, the base having a recess within which to mount the pod in a releasable manner, the pod operable at a distance from the base, (c) a communications link between the pod and the base to transfer the patient data from the pod to the base, the base having a display area to visually display the patient data, (d) the recess can releasably hold a power supply for the base, (e) the recess is adapted to mount different sizes of pods with at least one pod being secured to a latching assembly, (f) the latching assembly has a pair of guide ribs in the recess to receive the pod and control the pod's motion in both the horizontal and vertical direction, (g) the guide ribs align the pod during insertion into the recess to ensure an electrical connection between a base interface connector and a pod interface connector that together provide the communications link, (h) the guide ribs of the latching assembly align a pod interface connector with a base interface connector to establish the direct electrical connection, (i) the base includes inserts to attach at least one of defibrillation paddles, a carrying bag, and a pod mounting bracket that holds the pod, (j) the base provides power to charge a battery that powers the pod, (k) the base provides charging power to the pod wirelessly, and (1) the cardiac therapy module synchronizes the electrical cardiac therapy to the patient's intrinsic rhythm based on the patient data.

A modular cardiac therapy system in embodiments of the invention may include one or more of the following features: (a) a base containing a cardiac therapy module adapted to deliver an electrical cardiac therapy to a patient, (b) a pod having a patient parameter module with patient lead cables attachable to the patient to collect patient vital signs, the pod operable at a distance from the base, (c) a communications link between the pod and the base to carry the patient vital signs from the pod to the base, the base having a monitor area to visually display the patient vital signs, and (d) a docking station to house the base in a releasable manner, the base operable when housed by the docking station or at a distance from the docking station.

A modular cardiac therapy system in embodiments of the invention may include one or more of the following features: (a) a base containing a cardiac therapy module adapted to deliver an electrical cardiac therapy to a patient, (b) a pod having a patient parameter module with patient lead cables attachable to the patient to collect patient data, the pod operable at a distance from the base, the cardiac therapy module in the base delivering an electrical cardiac therapy to the patent based on the patient data, (c) a communications link between the pod and the base to carry the patient vital signs from the pod to the base; (d) a docking station to house the base in a releasable manner, the base operable at a distance from the docking station, (e) the docking station houses the pod in a releasable manner, (f) the base mounts the pod in a releasable manner, (g) the docking station provides power to recharge batteries within the base and power the base, (h) the docking station provides power to recharge a battery within the pod, (i) the docking station comprises a restraining plate to secure the base thereto, (j) the restraining plate is coupled to a backing plate configured for being secured to a mounting surface, (k) the restraining plate is rotatable towards the backing plating for compact storage when not in use, (1) the docking station further comprises a blade extending vertically from the restraining plate into a recess defined in a lower surface of the base to secure the base to the restraining plate, and (m) a lever rotates the blade inside the recess to secure the base to the plate and enable electrical connection between the base and the docking station.

A modular external defibrillator system in embodiments of the invention may include one or more of the following features: (a) a base containing a defibrillator module adapted to deliver a defibrillation shock to a patient, the base containing a removable battery to source the power for the defibrillation shock, (b) a pod having a patient parameter module with patient lead cables attachable to the patient to collect patient vital signs, the pod operable at a distance from the base, the pod containing a removable battery to source the power to collect patient vital signs, the pod battery and the base battery being interchangeable between the base and the pod, (c) a communications link between the pod and the base to carry the patient vital signs from the pod to the base, the base having a monitor area to visually display the patient vital signs, (d) the base contains two removable batteries, and each base battery being interchangeable with the pod battery, (e) the base is connected to a printer to print out the patient data, and (f) the base includes printer to print out the patient data.

### BRIEF DESCRIPTION OF DRAWINGS

FIG. 1 is a pictorial representation of an external defibrillator having a patient module with a defibrillator/monitor in an embodiment of the present invention;
FIG. 2 is a pictorial representation of a latching assembly on a defibrillator/monitor in an embodiment of the present invention;
FIG. 3 is a pictorial representation of a mating assembly on a defibrillator/monitor in an embodiment of the present invention;
FIG. 4 is a pictorial representation of a mating assembly having a tethered connector in an embodiment of the present invention;

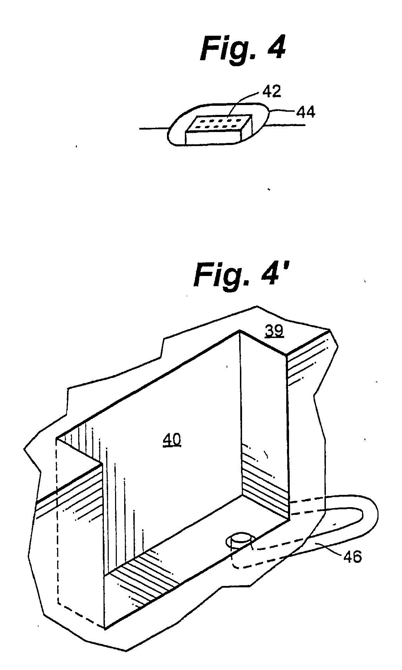

FIG. 4' is a pictorial representation of a tethered connector as shown in FIG. 4;

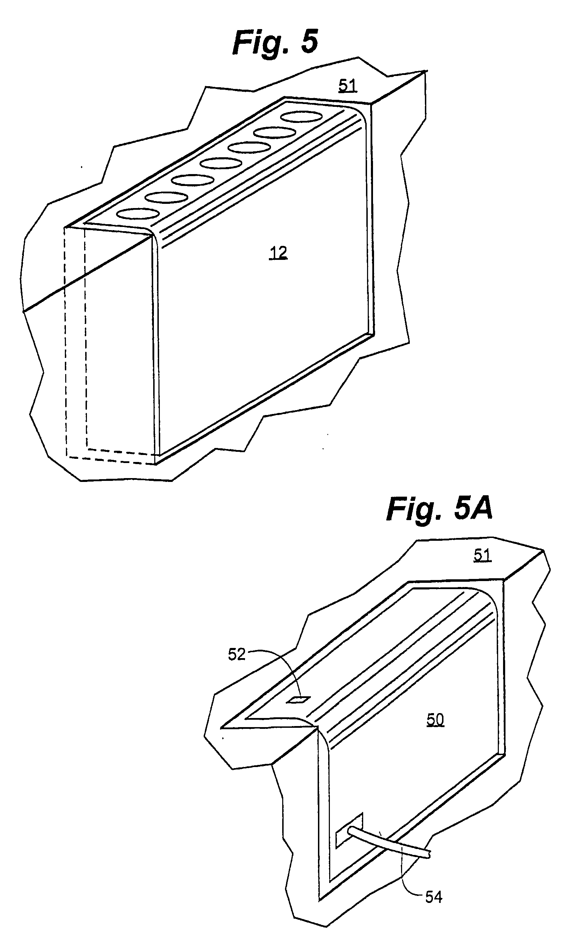

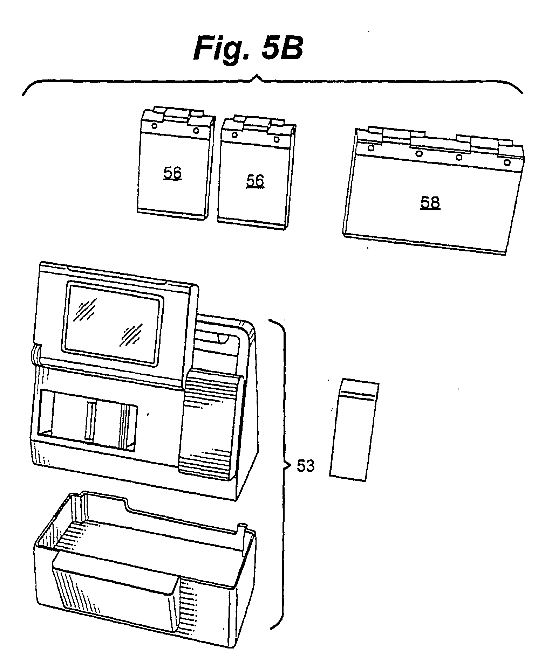

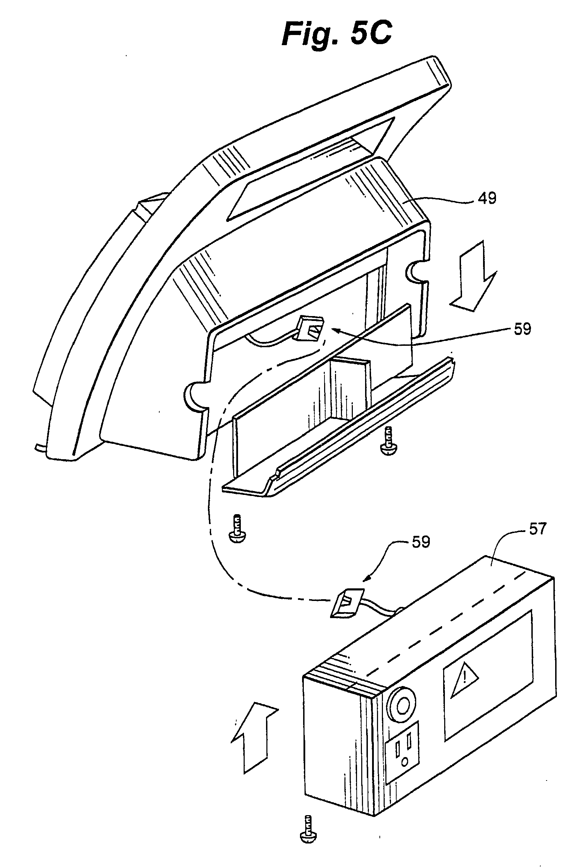

FIG. 5 is a pictorial representation of a defibrillator/monitor base in an embodiment of the present invention;
FIG. 5A is a pictorial representation of an alternate use for a defibrillator/monitor base in an embodiment of the present invention;
FIG. 5B is a front profile view of a defibrillator/monitor base providing an alternate power supply option in accordance with an embodiment of the present invention;
FIG. 5C is a view of a defibrillator/monitor providing an alternate power supply option in accordance with an embodiment of the present invention;

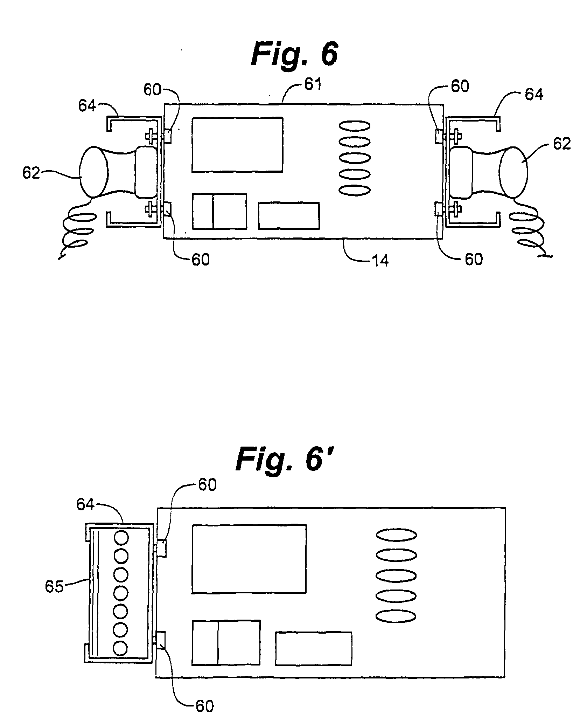

FIG. 6 is a pictorial representation of storage assembly for a defibrillator/monitor in an embodiment of the present invention;
FIG. 6' is a pictorial representation of storage assembly for a defibrillator/monitor in an embodiment of the present invention;

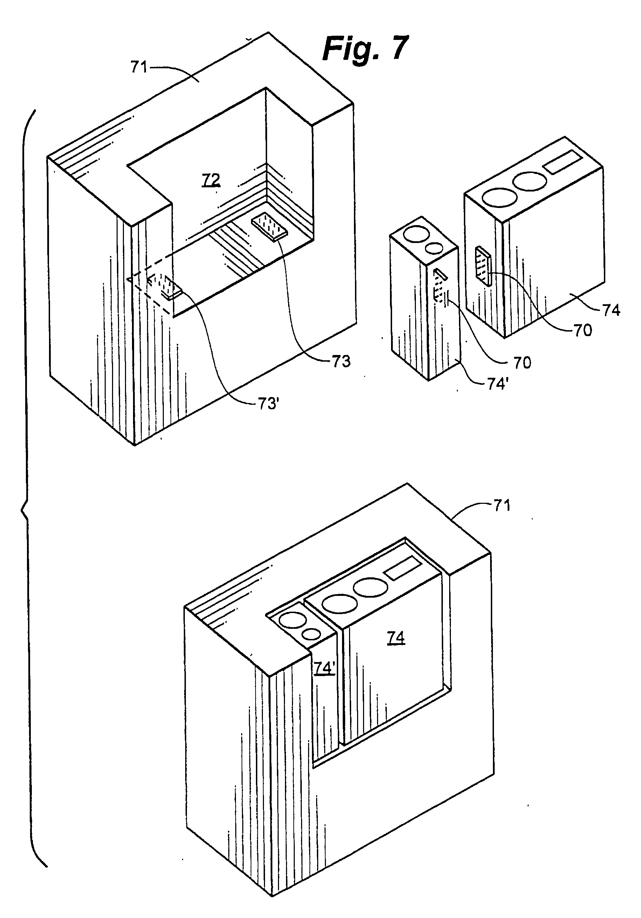

FIG. 7 is a pictorial representation of a multiple patient module storage and attachment assembly in an embodiment of the present invention;

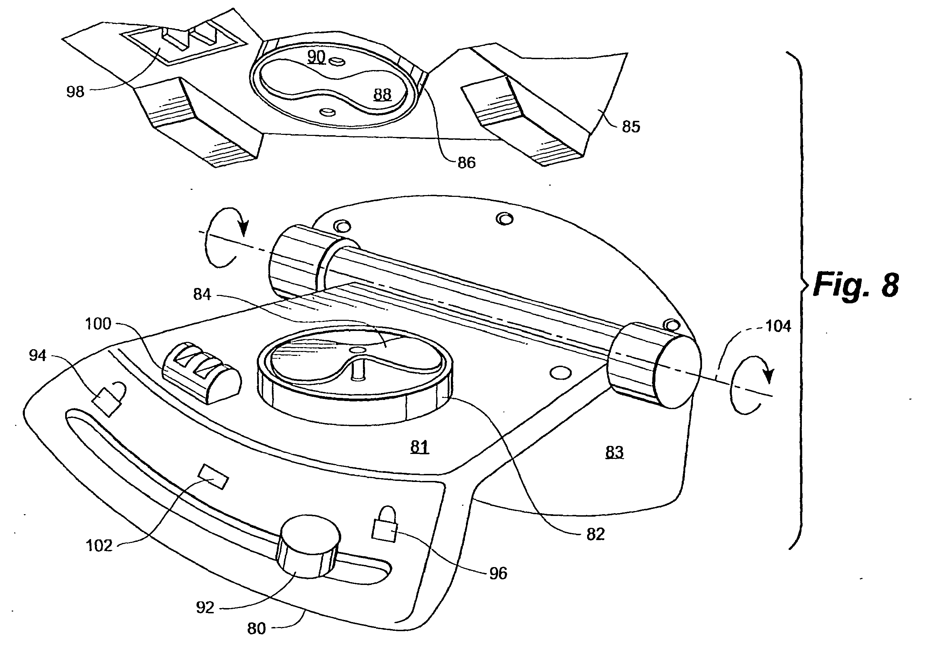

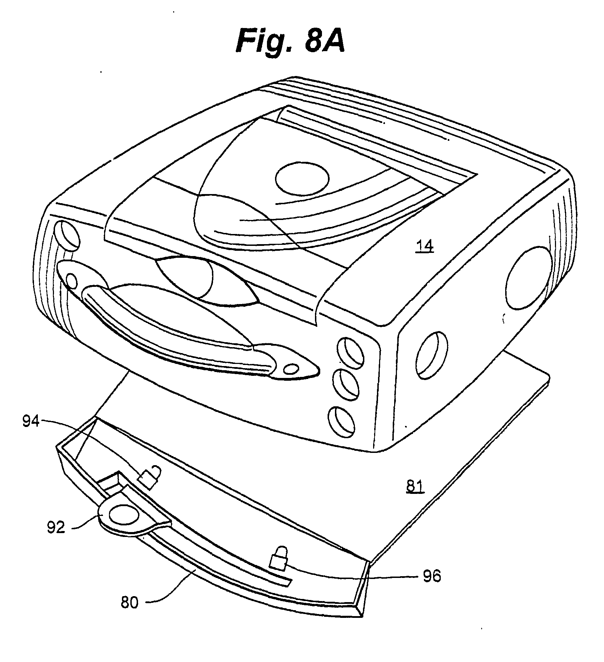

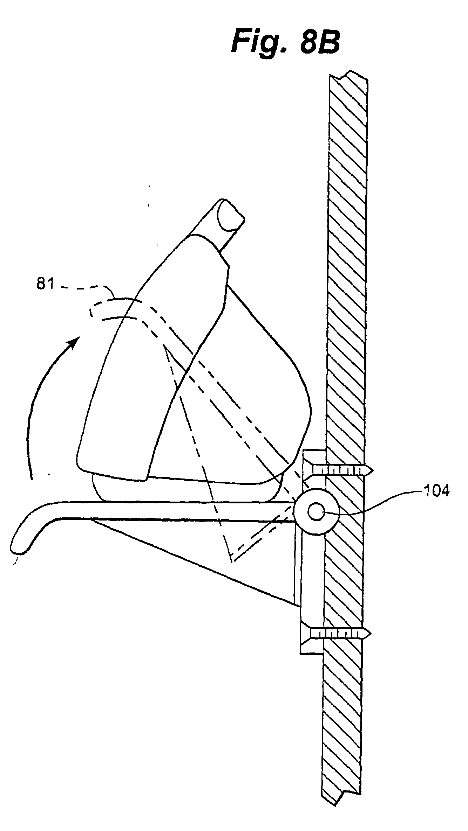

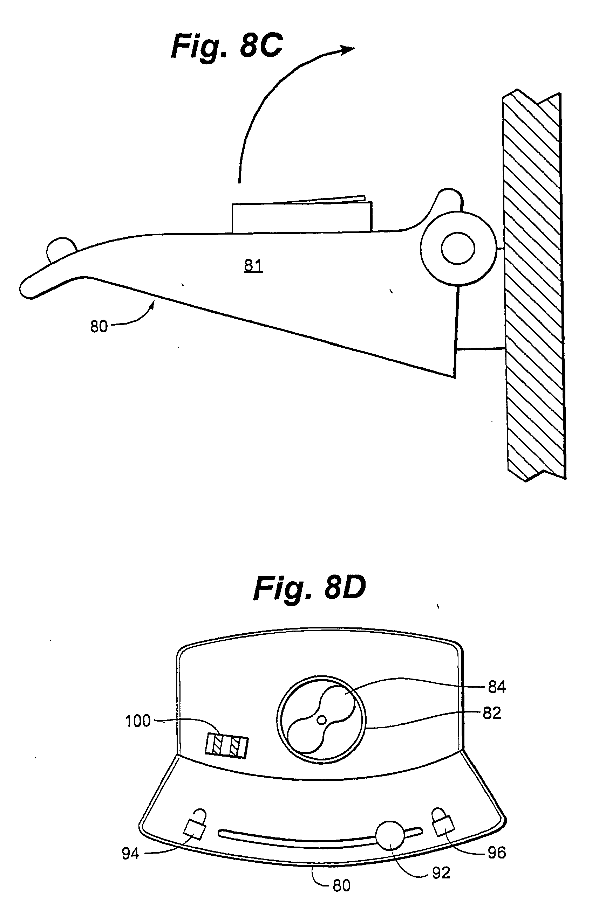

FIG. 8 is a pictorial representation of a docking station for a defibrillator/monitor in an embodiment of the present invention;
FIG. 8A is a pictorial representation of a docking station and defibrillator/monitor as shown in FIG. 8;
FIG. 8B is a side profile view of a docking station as shown in FIG. 8;
FIG. 8C is another side profile view of a docking station as shown in FIG. 8;
FIG. 8D is a top profile view of a docking station as shown in FIG. 8;

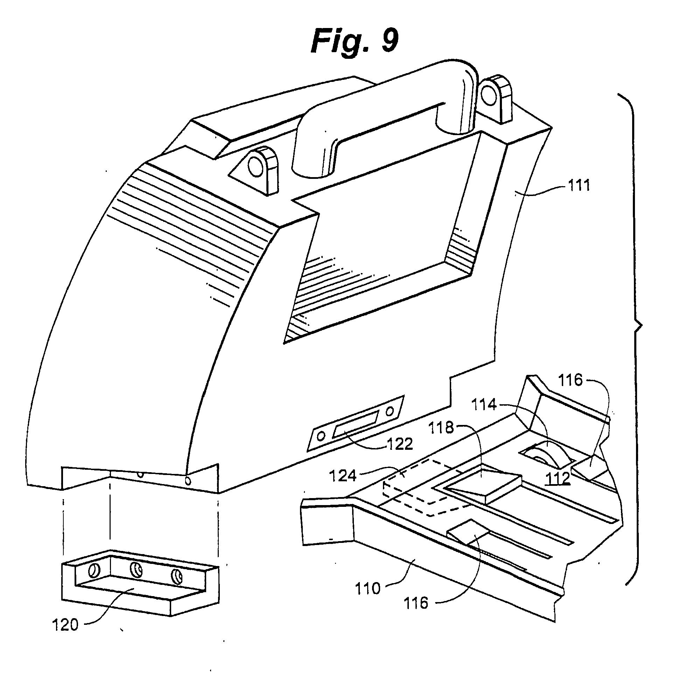

FIG. 9 is a side rear profile view of a docking station for a defibrillator/monitor in an embodiment of the present invention;

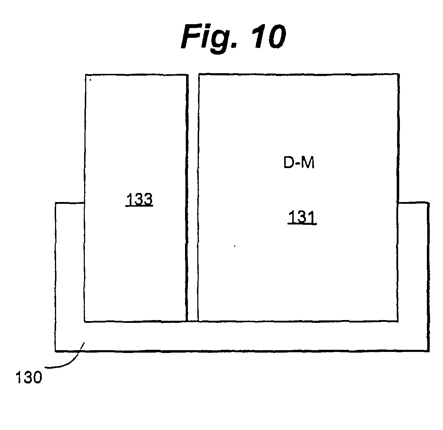

FIG. 10 is a front pictorial of a docking station for a defibrillator/monitor and patient module in an embodiment of the present invention;

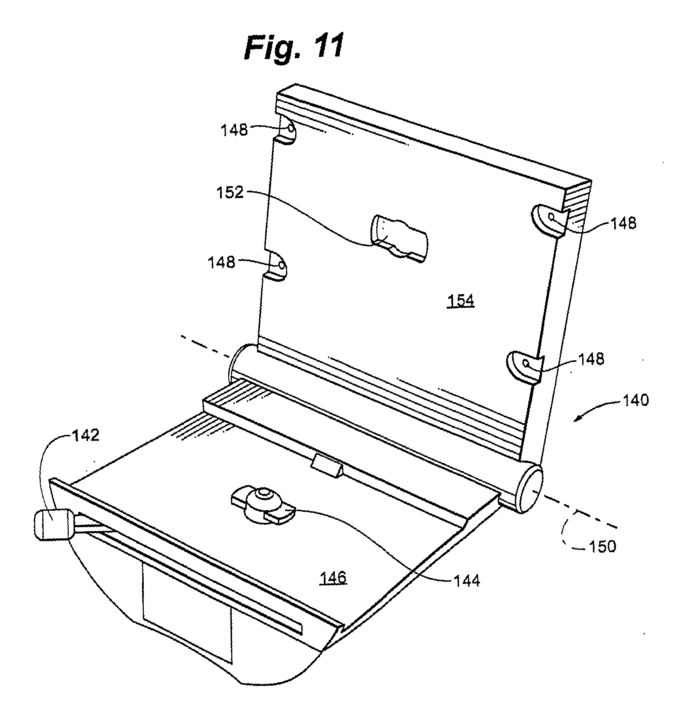

FIG. 11 is a front profile view of a docking station for a defibrillator/monitor in an embodiment of the present invention;

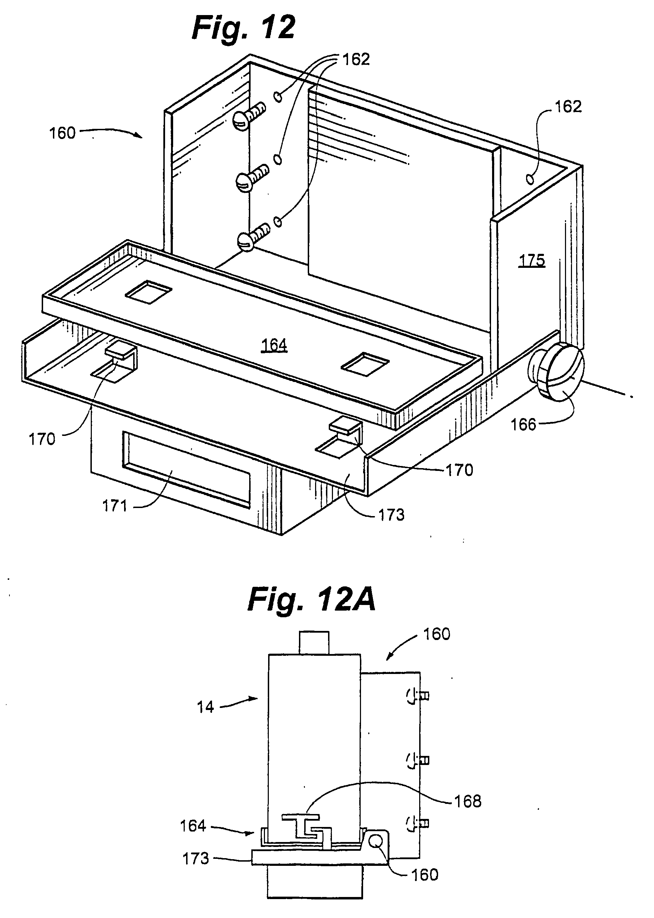

FIG. 12 is a front profile view of a docking station for a defibrillator/monitor in an embodiment of the present invention;
FIG. 12A is a side profile view of a docking station of Fig. 12;

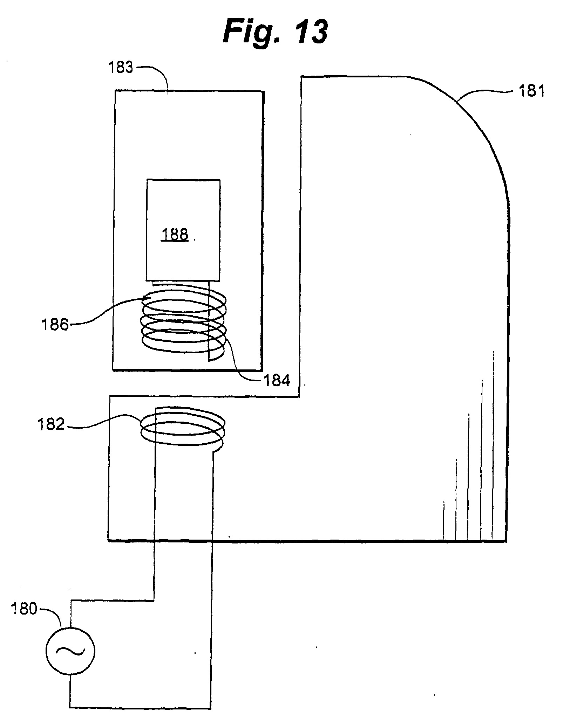

FIG. 13 is a side profile schematic of a defibrillator/monitor and a patient module according to a patient module wireless battery charging embodiment of the present invention;

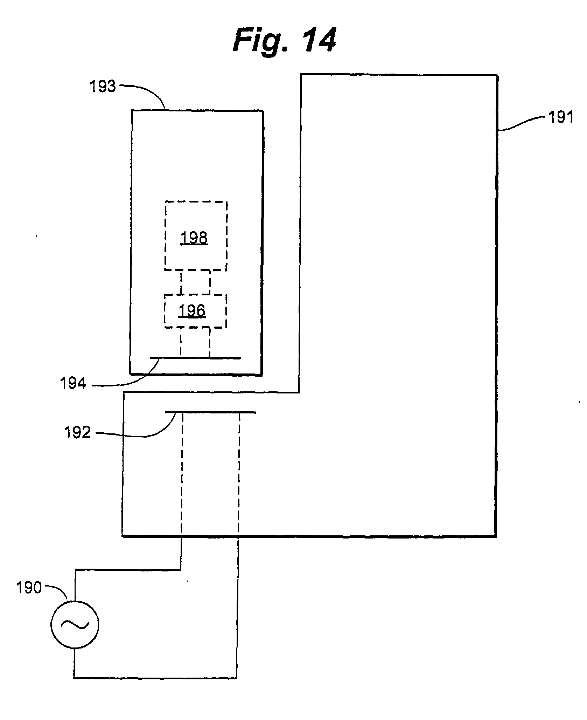

FIG. 14 is a side profile schematic of a defibrillator/monitor and a patient module according to a patient module wireless battery charging embodiment of the present invention;

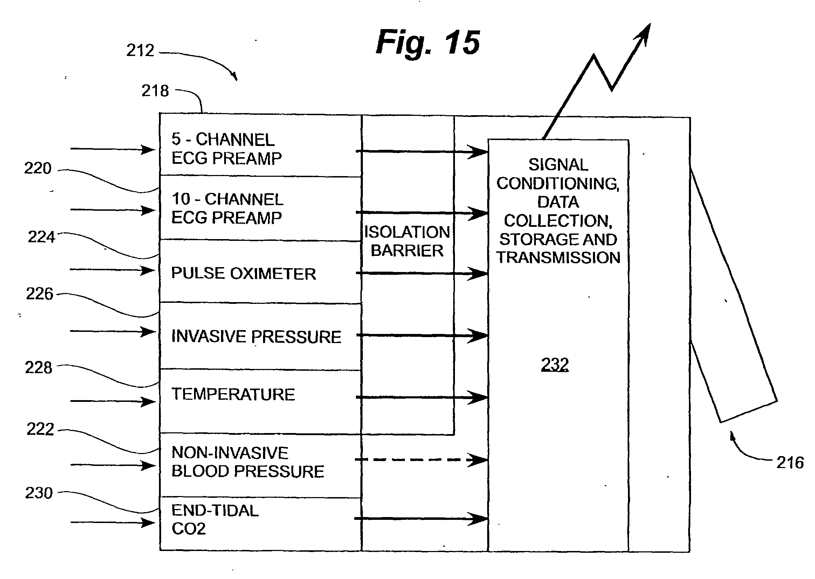

FIG. 15 is an upper level pictorial representation of a patient module in an embodiment of the present invention;

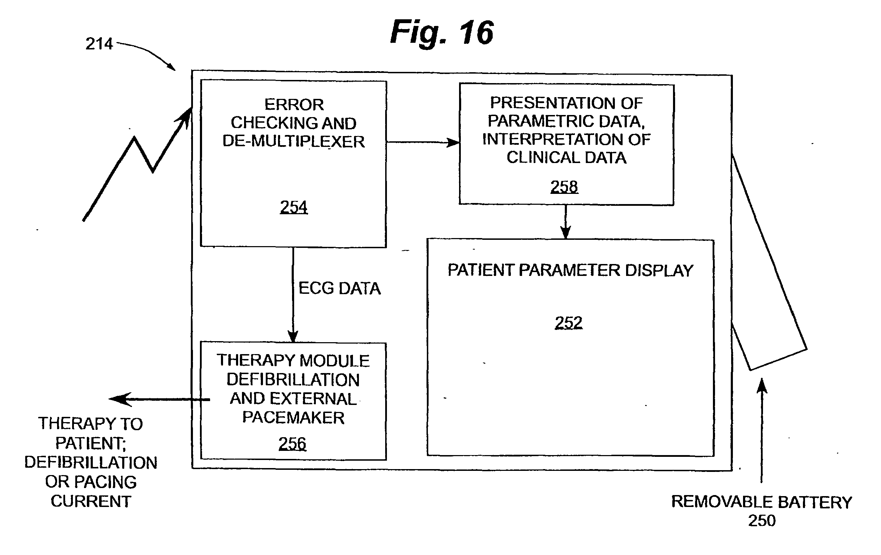

FIG. 16 is an upper level pictorial representation of a defibrillator/monitor in an embodiment of the present invention;

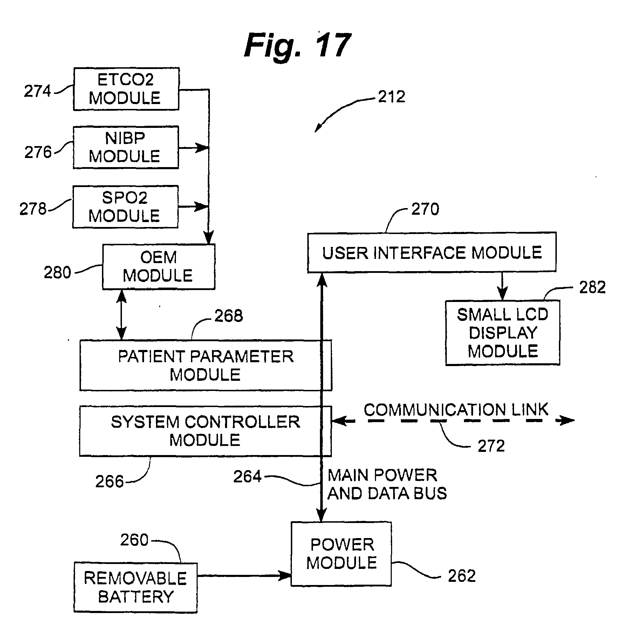

FIG. 17 is a schematic view of a patient module in an embodiment of the present invention; and

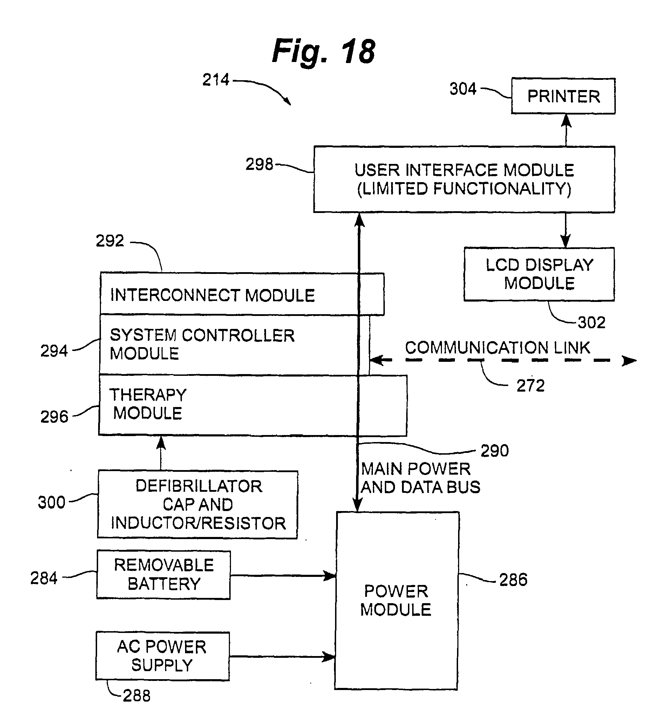

FIG. 18 is a schematic view of a defibrillator/monitor in an embodiment of the present invention.

### DETAILED DESCRIPTION

The following detailed description is to be read with reference to the figures, in which like elements in different figures have like reference numerals. The figures, which are not necessarily to scale, depict selected embodiments and are not intended to limit the scope of the invention. Skilled artisans will recognize the examples provided herein have many useful alternatives falling within the scope of the invention.

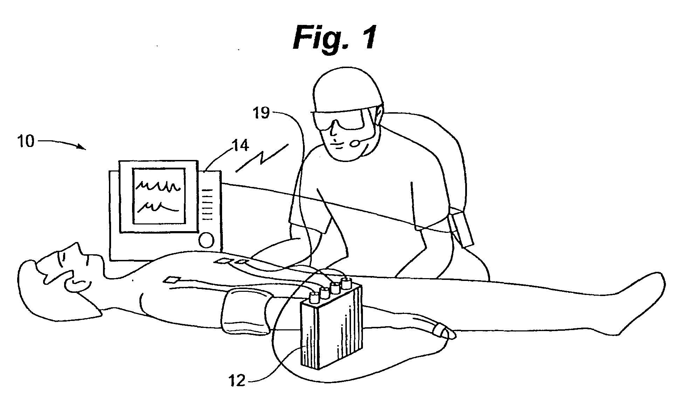

With reference to Figure 1, a pictorial representation of an external defibrillator having a patient module with a defibrillator/monitor in an embodiment of the present invention is shown. External defibrillator 10 is comprised of two components the patient module (pod) 12 and the defibrillator/monitor (base) 14, which communicate patient data (e.g., vital signs) wirelessly and share common replaceable battery technology. Pod 12 generally rests within base 14, generally in the back of base 14 as will be discussed in more detail below. The user, during an emergency, has the option of carrying base 14 with pod 12 attached or simply carrying pod 12 to the emergency site. Since pod 12 is smaller and lighter than base 14, generally it will be easier for the user to simply carry pod 12. By carrying pod 12, the user is free to carry more ALS equipment and not be slowed by the heavier and more awkward base 14.

As shown in Figure 1, pod 12 connects to patient via several leads 19 in order to measure the patient's vital signs. The pod communicates the patient's vital signs wirelessly to defibrillator monitor 14. The patient data or vital signs collected may include 3, 4, and 5 lead ECG readings, 12 lead ECG readings, non-invasive blood pressure (NIBP), pulse oximeter data, capnography and other respiratory data, invasive blood pressure, body temperature, CO₂ levels, and additional patient monitoring functions. Additionally, pod 12 may include a small display (not shown) replicating some or all of the information such as waveforms, numerical data, and vital signs being transmitted to base 14.

Base 14 includes a therapy module and therapy cables. The therapy module has the capability to provide therapeutic functions such as pacing, defibrillation or synchronous cardioversion without attaching another monitor/defibrillator to the patient. The therapy cables typically include patient paddles or electrodes that attach between the patient and the base 14 in order to deliver the therapy to the patient. Since pod 12 connects to the patient and transmits vital signs to the base 14, then base 14 need not also have patient monitoring cables. Accordingly, paramedic mobility and ease of use are greatly increased. The defibrillator in the base 14 may be configurable in either an ALS mode or an AED mode. The ALS mode includes a multi-parameter monitoring capability and all of the defibrillator therapy delivery capability. Additionally the base unit may be just as an AED.

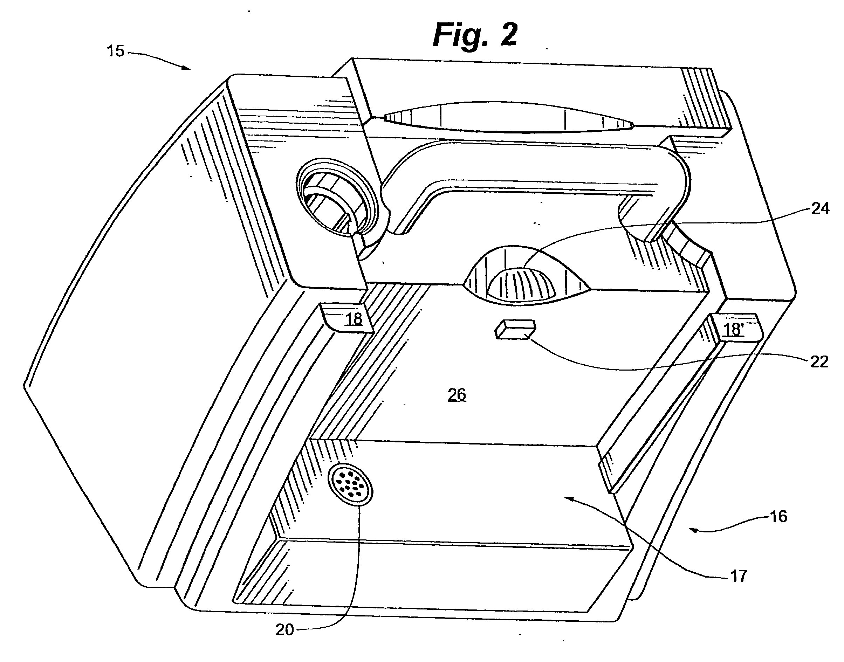

Pod 12 includes some means by which it can be attached to base 14 for the purpose of carrying base 14 to an emergency scene. With reference to Figure 2, a pictorial representation of a latching assembly on a defibrillator/monitor in an embodiment of the present invention is shown. Latching assembly 16 is used to attach pod 12 to base 15. Latching assembly 16 is provided with guide ribs 18 and 18', which provide control motion in both the horizontal and vertical direction, aligning base-to-pod interface connector 20 with a similar connector (not shown) on pod 12. Latch 22 actuates automatically when pod 12 is placed within slot 17. When pod 12 is lowered within slot 17, latch 22 will align with a matching cavity on pod 12 to hold pod 12 within slot 17. When the user wants to remove pod 12 from slot 17, they simply press button 24, which pushes the spring-loaded latch 22 back within rear wall 26 of slot 17. Pod 12 is released and the user simply pulls pod 12 from slot 17. It is further contemplated pod 12 could be spring released by springs placed at the base of ribs 18 and 18' or perhaps a spring placed within base-to-pod connector 20. It is also further contemplated base-to-pod connector could be most any type of connector such as a USB port, an AC power connector, an RS-232 connector or any other type of connector known to those skilled in the art. In addition, it is contemplated pods of different sizes could be used within slot 17. For example, a large pod would be guided in place with ribs 18 and 18' and held with latch 22. If a smaller pod were being used, then the smaller pod would be guided in place with rib 18 so pod 12 aligns with base-to-pod connector 20 and held in place with latch 22.

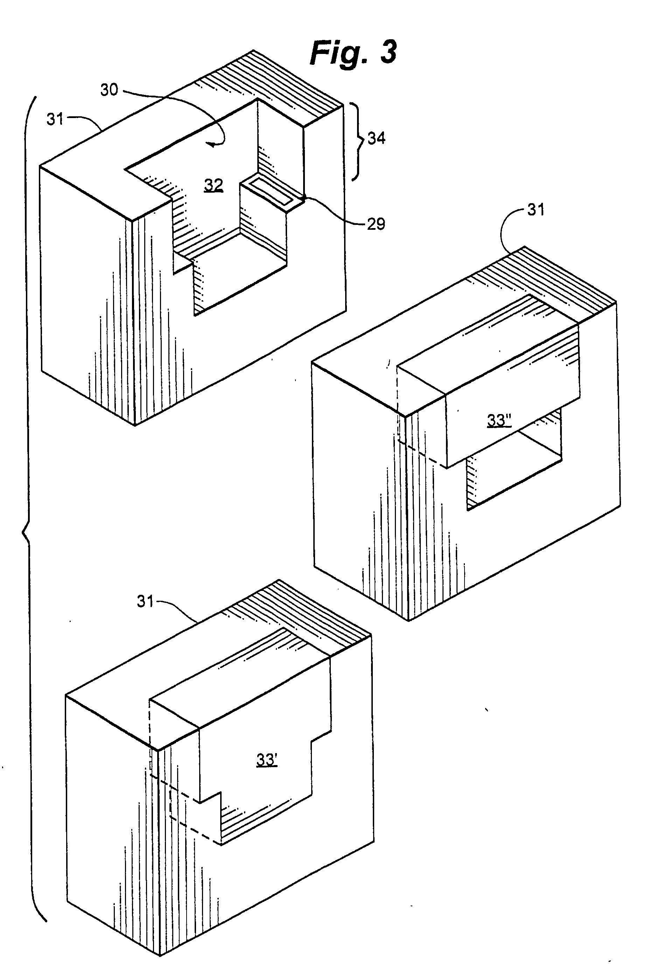

With reference to Figure 3, a pictorial representation of a mating assembly on a defibrillator/monitor in an embodiment of the present invention is shown. Mating assembly 30 comprises recess or slot 32 which can house two types of pods 33' and 33". Since both pods 33' and 33" have the same dimension in the horizontal, both pods 33' and 33" are capable of fitting within slot 32. When large pod 33' is fit within slot 32 it takes up generally all the available room within slot 32. When small pod 33" is placed within slot 32 only the room within upper portion 34 is taken up. Both pod 33' and 33" are held in place by attachment to base-to-pod connector 29. It is contemplated, however, a latch assembly similar to that of Figure 2 could be utilized to ensure pods 33' and 33" remain within slot 32.

With reference to Figure 4 and 4', a pictorial representation of a mating assembly having a tethered connector in an embodiment of the present invention is shown. In this embodiment, a pod similar to 12 rests within slot 40 and connects to base-to-pod connector 42, which allows base 39 and a pod to communicate with each other. Base-to-pod connector 42 rests freely within connector cavity 44, which allows connector cable 46 to retractably exit and enter base 39 as shown in Figures 4 and 4'. Tethered cable 46 allows a pod to mate with and rest within base 39 or mate with base 39 when not docked within slot 40. It is sometimes preferred that base 39 communicate with a pod through tethered cable 46 since communications through a direct connection is generally faster as is discussed in more detail below. This is especially the case in the present embodiment as base 39 is equipped with a USB bus, which provides quick communication of information between a pod and base 39. Base 39 is also able to automatically detect when tethered cable 46 is plugged in so direct communications can be established immediately. A direct communication between a pod and base 39 can be established. This automatic establishment of direct communication between a pod and base 39 includes when a pod is docked within base 39 and a connection is made between a pod and base 39 through connector 42.

Generally base 39 and a pod communicate wirelessly to assist in preventing the tangling of cables, which can occur between a patient and base 39, particularly when transporting patients. Tethered cable 46 provides a back-up system for use when the wireless link between pod 12 and base 14 fails for whatever reason or when precise signal synchronization demands a wired connection. Tethered cable 46 also provides the added advantage in that the user cannot lose cable 46 because it is tethered to base 39. Similar to the discussion above, wireless links can impose a delay in communication between a pod and base 39 longer than may be experienced with a cable. When communications between base 39 and a pod require a faster response time (such as application of synchronous cardioversion or pacing where information from a pod must be transmitted to base 39), the user is advised of the need to plug cable 46 into the pod. The user is provided a user interface message to inform them of the need to attach cable 46.

With reference to Figures 5 and 5A, a pictorial representation of an alternate use for a defibrillator/monitor base 51 in an embodiment of the present invention is shown. As discussed above, typically a pod 12 is placed within a base using components such as a latching assembly and mating assembly when the pod is not in use or when base 51 is carried to an emergency site, as shown in Figure 5. As an alternate use of a base 51, the embodiment of Figure 5A allows for an AC power supply 50 to be placed within the base and to provide power to base 51. Power supply 50 would transfer power to base 51 through a base-to-pod connector (not visible) similar to connector 20. Upon power supply 50 being plugged into a wall outlet via power cord 54, power LED 52 provides an indication to the user notifying them power supply 50 is powering 51 and/or charging the base's battery. Power supply 50 is typically used when for example; a pod is being used on a patient such as on a gurney or next to the patient to provide constant power and reduce battery depletion. Power supply 50 could also be used when the user desires to substantially power base 39 through line power. Thus an alternate pod mounting device would have to be provided as will be discussed in more detail below.

With reference to Figure 5B, a front profile view of a defibrillator/monitor providing an alternate power supply option in accordance with an embodiment of the present invention is shown. Figure 5B shows a modular integrated defibrillator/monitor 14 with multiple power supply options. However, unlike the embodiment of Figure 5A, the present embodiments are able to house pod 12 and provide for an alternate power supply. In Figure 5B, base 53 typically is powered by dual batteries 56. In the alternative, base 53 could be powered by A/C power module 58. In this embodiment, batteries 56 are replaced with A/C power module 58. Module 58 is then connected to A/C power to power base 53 without having to remove the pod. In another embodiment shown in Figure 5C, base 49 has a removable bottom section 59 able to accommodate A/C power module 57. Therefore, base 49 is able to accommodate a pod and an alternate power supply.

With reference to Figures 6, and 6', a pictorial representation of storage assembly for a defibrillator/monitor in an embodiment of the present invention is shown. Figure 6 shows base 61 with brass inserts 60 mounted on the side of base 60. Brass inserts 60 can be used as clips to attach hand paddles 62, or side-mounted carrying bags, or a bracket 64 to side mount pod 65. Bracket 64 allows defibrillator 10 the ability to carry various types of defibrillator support equipment. Further, as stated above, the user has the ability to mount a pod outside of its docking assembly so a power supply 50 can be within the docking assembly and the base can be powered from line power as described above. This alternate mounting assembly provides the advantages of providing easily accessible connectors for troubleshooting and easier access for connection and disconnection of various leads and connectors.

With reference to Figure 7, a pictorial representation of a multiple patient module storage and attachment assembly in an embodiment of the present invention is shown. Pods can come in different sizes generally representing the capability of the pod. For example, smaller pod 74' would provide only the basic features for an external defibrillator, while medium pod 74 would provide several additional features. In the present embodiment, pods 74 and 74' can be docked together in mounting recess or slot 72 contemporaneously. In one embodiment, pod 74 could be latched within mounting slot 72 communicating with base 71 through connector 73. Similarly, pod 74' can be placed within mounting slot 72 contemporaneously with pod 74 and latched in a communicating relationship with base 71 through connector 73'. In another embodiment, pods 74 and 74' could be placed within mounting slot 72 without the need for two base-to-pod connectors 73. In the embodiment, pod 74 and 74' latch together and communicate through connectors 70. Then both pods 74 and 74' are placed within mounting slot 72 and latched in a communicating relationship with base 71 through connector 73. This embodiment not only limits the amount of connectors needed on base 71, but also allows the user to choose the amount of functions the pod can perform. For example, if the user simply needed to perform an ECG, then the user could choose to carry small pod 74'. However, if the emergency situation required additional functions such as monitoring blood pressure in a non-invasive method or a pulse oximeter, then the user would choose to carry medium pod 74'. In addition, if the emergency situation required all of the available pod functions, then pod 74' could be latched together with pod 74 to provide a large pod having all necessary functions.

With reference to Figures 8, 8A, 8B, 8C, and 8D a pictorial representation of a docking station for a defibrillator/monitor in an embodiment of the present invention is shown. Docking station 80 performs two main roles. It restrains base 85 under semi-violent maneuvers (2-5G's) and provides DC power to charge the batteries (not shown) and operate base 85. Docking station 80 is comprised of restraining plate 81 held to a wall by backing plate 83. It is contemplated restraining plate 81 could be attached to any surface such as a horizontal shelf of a vertical wall. Restraining plate 81 provides a ring 82 housing a self-aligning propeller or blade 84 as best seen in Figure 8D. When the user desires to dock base 85 as shown in Figure 8A, it is placed on restraining plate 81 where recess 86 fits over ring 82 and blade 84 fits within opening 88 in plate 90. When base 85 is properly placed on restraining plate 81, the user slides lever 92 from unlocked position 94 to locked position 96. Blade 84 has a quarter turn twist which pulls base 85 to restraining plate 81 when the user slides lever 92 from unlock position 94 to locked position 96. As lever 92 moves towards locked position 96 electrical power connection 98 will mate with power connection 100 as base 85 is pulled closer to restraining plate 81. When connectors 98 and 100 make a good electrical contact, indicator 102 illuminates informing the user a good electrical connection has been made between base 85 and docking station 80. It is of note that no power is applied to connector 100 until a closed circuit connection is made with connector 98. Therefore, if base 85 is not docked at docking station 80, then there is no power applied at connector 100. It is contemplated when lever 92 is in locked position 96, a short electrical pulse is sent to connector 100 to verify it is in electrical contact with connector 98.

When base 85 is locked to restriction plate 82 docking station 80 provides power to base 85. When in locked position 96, docking station 80 restricts the base's up and down, side to side movement to prevent damage to base 85. It is contemplated docking station 80 could also dock a pod. It is further contemplated docking station 80 could also provide communications from base 85 to a network, such as is described in commonly owned U.S. Patent Application No. 10/378,001 filed February 28, 2003 titled "Medical Device Status Information System".
Finally, when the user has removed base 85, restriction plate 81 quickly rotates out of the way for compact storage along axis 104 as more clearly shown in Figure 8B.

With reference to Figure 9, a side rear profile view of a docking station for a defibrillator/monitor in an embodiment of the present invention is shown. Docking station 110 is comprised of sliding plate 112, rollers 114, ribs 116, and latch 118. In use, base 111 is modified with guides 120 held in place by screw, bolts or the like, which slide under ribs 116 when base 111 is placed upon and slid on sliding plate 112. Rollers assist in sliding base 111 along sliding plate 112. When base 111 is fully within docking station 110, latch 118 engages a notch on the underside of base 111, which prevents base 111 from exiting sliding plate 112. When guides 120 are within ribs 116, base 111 is unable to move from side to side. Thus latch 118 in combination with guides 120 and ribs 116 prevent any substantial movement of base 111. Further, when base 111 is fully within docking station 110, connector 122 mates with another connector (not shown) at the rear of docking station 110, which provides power to run base 111 and charge the base's battery as well. When the user chooses to remove base 111 from docking station 110, they would simply press spring loaded button 124, which releases latch 118 and allows for easy removal of base 111.

With reference to Figure 10, a front pictorial of a docking station for a defibrillator/monitor and patient module in an embodiment of the present invention is shown. In the present embodiment, docking station 130 houses pod 133 and base 131. It is contemplated docking station 130 could be similar to the structure of docking stations 80 or 110 adjusting of course the size of the docking station to accommodate pod 133 and base 131. In this embodiment, both pod 133 and base 131 are held securely in docking station 130 and both pod 133 and base 131 are provided with power to charge each respective battery and power each respective device. It is further contemplated pod 133 could be an alternate in the event the pod within base 131 failed. Therefore, in the event of a pod failure, the user would simply return to docking station 130 and retrieve pod 133 place it within the base's docking station (or connect to base 131 through a tethered cord) where base 131 would automatically identify pod 133 and dynamically pair up with pod 133.

With reference to Figure 11, a front profile view of a docking station for a defibrillator/monitor in an embodiment of the present invention is shown. Similar to the docking station of Figure 8, the present docking station 140 has a locking handle 142 a propeller or blade 144, and restraining plate 146. A base would rest on restraining plate 146, the user would then slid handle 142 into the locking position, thus rotating propeller 144 to hold base 14 to restraining plate 146. Docking station 140 is held to a wall by screws, bolts, or the like through retaining holes 148. When the user removes the base by taking locking handle 142 to the unlock position and lifting the base, restraining plate 146 is moved upward along axis 150 until it rests against back plate 154. The user then moves locking handle 142 into the locking position, which causes propeller 144 to engage aperture 152 and thus retain restraining plate 146 to back plate 154 thus keeping docking station 130 out of the way for others who may be walking by.

With reference to Figures 12 and 12A, front and side profile views of a docking station for a defibrillator/monitor in an embodiment of the present invention is shown. Docking station 160 is attached to a wall by screw, bolts, or the like through retaining holes 162. Base 14 is placed upon tray 164 and then the user would turn locking knob 166 to the locking position. By turning locking knob 166 base 14 is pulled back towards the wall until hook 168 on base 14 engages hook 170 on support 173. Battery 171 provides power to the base and can recharge the battery if the base carries a rechargeable battery. This allows docking station 160 to be used in an area when line power is inaccessible. When the base is removed from docking station 160, support 173 is lifted toward wall mount 175 for storage.

With reference to Figure 13, a side profile schematic of a defibrillator/monitor and a patient module according to a patient module wireless battery-charging embodiment of the present invention is shown. In the present embodiment, base 181 is able to charge pod battery 188 wirelessly from line power 180 through primary coil 182 located in base 181 and secondary coil 184 located in pod 183. Bridge rectifier 186 acts to convert A/C line power 180 to a D/C voltage which charges battery 188 of pod 183. This concept can even be extended to cover a docking station wirelessly charging a base unit as is disclosed in commonly owned U.S. Patent Application titled "Apparatus and Method for Maintaining a Defibrillator Battery Charge and Optionally Communicating" serial number 10/423,805 filed on April 15, 2003.

With reference to Figure 14, another side profile schematic of a defibrillator/monitor and a patient module according to a patient module wireless battery charging embodiment of the present invention is shown. In this embodiment, proper alignment of a first plate 192 connected to line power 190 within base 14 and a second plate 194 within pod 193 provides for capacitive coupling. As before, bridge rectifier 196 acts to convert A/C line power 190 to a D/C voltage which charges battery 198 of pod 193.

With reference to Figure 15, an upper level pictorial representation of a patient module in an embodiment of the present invention is shown. Generally, pod 212 uses replaceable or rechargeable batteries 216 for power and comprises any combination of the following features: 3, 4, and 5 lead ECG inputs 218, 12 lead ECG inputs 220, non-invasive blood pressure (NIBP) input 222, pulse oximeter input 224, capnography input (not shown), invasive blood pressure input 226, temperature input 228, CO₂ input 230, additional patient monitoring functions, wireless (RF) transceiver 232 to transmit any or all real time patient data to base 214. Additionally, pod 212 may include a small display (not shown) replicating some or all of the information such as waveforms, numerical data, and vital signs being transmitted to base 214. Additionally, pod 212 includes some means by which it can be attached to base 214 for the purpose of carrying base 214 to an emergency scene as is discussed in detail above.

With reference to Figure 16, an upper level pictorial representation of a defibrillator/monitor in an embodiment of the present invention is shown. Base 214 uses a replaceable or rechargeable battery 250 for power. Batteries 216 and 250 are generally similar in battery chemistry, electrical, and mechanical features to permit the interchangeability between batteries 216 and 250. Additionally, base 214 comprises a display 252 sufficient to show current and historical patient data, a transceiver (not shown) to send acquired patient data onto a receiving station or third party data receiver (discussed in more detail below), a module 256 to synchronize shocks and pacing pulses to the patient's intrinsic rhythm from data acquired by a pod 212, an error checking and demultiplexing module 254 receiving and processing data received from pod 212, and a data interpretation module 258 which analyzes data acquired by pod 212 and makes certain interpretive statements on the patient's cardiac or respiratory condition, displays vital sign trends, and provides additional functions found in ALS monitoring products.

With reference to Figure 17, a schematic view of a patient monitor in an embodiment of the present invention is shown. As discussed above, pod 212 can be powered from a removable/rechargeable battery 260. Power module 262 processes the incoming power into appropriate power levels for each of the internal components. Power module 262 routes the pod's power supply through main power and data bus 264 to system controller module 266, patient parameter module 268, and user interface module 270. As discussed above, pod 212 can be used wirelessly, however, pod 212 can be directly connected through a tethered cable 46 or through attachment to a connector 20 to utilize the speed of data bus 264.

System controller module 266 controls interaction of all the pod's modules through data bus 264 and interaction with base 214 through wired or wireless (e.g., IrDA, RF, etc.) communication link 272 or through data bus 264 if pod 212 is connected to base 214. Patient parameter module 268 monitors functions such as invasive blood pressure, patient's temperature, and inputs from the pod leads. Module 268 further collects inputs from EtCO2 module 274, NIBP module 276, and SpO2 module 278 through OEM module 280. Patient parameter module 268 takes all of these inputs and processes them for display and routes only a limited number of inputs to Small LCD display module 282 through user interface module 270. User Interface module 270 allows the user to primarily interact with pod 212; however, it is contemplated that user could use the module 270 to interact with base 214 as well.

With reference to Figure 18, a schematic view of a defibrillator/monitor in an embodiment of the present invention is shown. Base 214 is powered by a removable/rechargeable battery 284, which provides power to power module 286. Alternatively, base 214 could be powered by A/C line power 288. Power module 286 processes the incoming power into appropriate powered levels for each of the internal components. Power module 286 also routes the bases power supply through main power and data bus 290 to interconnect module 292, system controller module 294, therapy module 296, and user interface module 298. Interconnect module 292 is utilized to detect how pod 212 is connected to base 214 (wirelessly, docked, or tethered cable). Similar to system controller module 266 (in Figure 17), system controller module 294 controls all interaction of all of the base's modules through data bus 290 and interaction with pod 212 through wired or wireless connection communication link 272 or through data bus 290 if pod 212 is connected to base 214. Therapy module 296 synchronizes shocks and pacing pulses to the patient's intrinsic rhythm from data acquired from pod 212. Module 296 administers shocks from voltages via the defibrillation cap 300 and, in turn, administers pacing pulses to a patient. User interface module 298 allows the user to primarily interact with base 214; however, it is contemplated that user could use the module 298 to interact with pod 212 as well. LCD module 302 allows the user to view a patient's monitored parameters. Finally, the user has the option to print out patient information on a printer 304 (e.g., a 100mm strip chart printer).

One skilled in the art will appreciate that the present invention can be practiced with embodiments other than those disclosed. The disclosed embodiments are presented for purposes of illustration and not limitation, and the present invention is limited only by the claims that follow.

## Claims

1. A modular external defibrillator system, comprising:
a base (14) containing a defibrillator module;
a pod (12) having a patient parameter module with patient lead cables attachable to a patient to collect at least one patient vital sign, the pod operable at a distance from the base, the pod and the base being adapted to be optionally coupled together to be carried as a unit and separable into separate units ; and
a wireless communications link between the pod and the base to carry the at least one vital sign from the pod to the base, the defibrillator module delivering a defibrillation shock to the patient based on the at least one vital sign received from the pod.

2. The defibrillator system of claim 1, wherein the communications link is provided by both a wireless communications link and a direct electrical connection between the pod and the base.

3. The defibrillator system of claim 1, wherein a direct electrical connection between the pod and the base serves as an alternate communications link to the wireless communications link.

4. The defibrillator system of claim 1, wherein the communications link includes a direct electrical connection between the base and two or more of the pods.

5. The modular external defibrillator system of claim 1, wherein the base has a monitor area to visually display the patient vital signs.

6. The defibrillator system of claim 1, wherein the patient vital signs monitored by the pod include one or more of multi-lead ECG data, non-invasive blood pressure data, pulse oximeter data, capnography data and respiratory data, invasive blood pressure readings, and patient temperature data.

7. The defibrillator system of claim 5, wherein the base monitor area visually displays one or more of multi-lead ECG data, non-invasive blood pressure data, pulse oximeter data, capnography data, invasive blood pressure readings, and patient temperature data.

8. The system of claim 1, wherein the defibrillator module synchronizes defibrillation shocks to the patient's intrinsic rhythm based on the patient vital signs.

9. The system of claim 1, wherein the base includes a data interpretation module which analyzes the patient vitals signs to form interpretive statements on the patient's cardiac or respiratory condition.

10. The system of claim 1, wherein the base has a latching assembly to mount the pod in a releasable manner, the pod operable at a distance from the base.

11. The system of claim 10, wherein the latching assembly has a recess to receive the pod.

12. The system of claim 11, wherein the recess can releasably hold one or more pods.

13. The system of claim 11, wherein the recess releasably mounts two of the pods.

14. The system of claim 13, wherein the two pods are latched together and communicate via a connector on each pod.

15. The system of claim 14, wherein the base includes separate connectors to communicate with each pod.

16. The system of claim 14, wherein the pods monitor different vital signs.

17. The system of claim 10, wherein the base has a plurality of base interface connectors to provide a direct electrical connection with a plurality of pods in the latching assembly.

18. The system of claim 17, wherein each pod interface connector can be aligned and connected to another pod's interface connector.

19. The system of claim 10, wherein the latching assembly further comprises a latch to hold the pod within the recess.

20. The system of claim 19, wherein the latch aligns with a cavity on the pod to hold the pod in the recess.

21. The cardiac therapy system of claim 19, wherein the latch is spring loaded.

22. The system of claim 19, wherein in the latching assembly further comprises a press button that releases the spring loaded latch when a user wants to remove the pod from the slot.

23. The system of claim 19, wherein the latching assembly further comprises at least one spring located within at least one guide rib.

24. The system of claim 19, wherein the latch is operably coupled to the base and aligns with a matching cavity on the pod.

25. The system of claim 1, wherein the base has a recess within which to mount the pod in a releasable manner, the pod operable at a distance from the base.

26. The system of claim 25, wherein the recess can releasably hold a power supply for the base.

27. The system of claim 25, wherein the recess is adapted to mount different sizes of pods with at least one pod being secured to a latching assembly.

28. The system of claim 25, wherein the latching assembly has a pair of guide ribs in the recess to receive the pod and control the pod's motion in both the horizontal and vertical direction.

29. The system of claim 28, wherein the guide ribs align the pod during insertion into the recess to ensure an electrical connection between a base interface connector and a pod interface connector that together provide the communications link.

30. The cardiac therapy system of claim 29, wherein the guide ribs of the latching assembly align a pod interface connector with a base interface connector to establish the direct electrical connection.

31. The system of claim 25, wherein the base includes inserts to attach at least one of defibrillation paddles, a carrying bag, and a pod mounting bracket that holds the pod.

32. The system of claim 25, wherein the base provides power to charge a battery that powers the pod.

33. The system of claim 32, wherein the base provides charging power to the pod wirelessly.

34. The system of claim 25, wherein the cardiac therapy module synchronizes the electrical cardiac therapy to the patient's intrinsic rhythm based on the patient data.

35. The system of claim 1, further comprising:
a docking station (8) to house the base in a releasable manner, the base operable when housed by the docking station or at a distance from the docking station.

36. The cardiac therapy system of claim 35, wherein the docking station houses the pod in a releasable manner.

37. The system of claim 35, wherein the docking station provides power to recharge batteries within the base and power the base.

38. The system of claim 35, wherein the docking station provides power to recharge a battery within the pod.

## Patentansprüche

1. Modulares externes Defibrillatorsystem mit:
einer Basis (14) mit einem Defibrillatormodul;
einer portablen Einrichtung bzw. Halterung (12) mit einem Patientenparametermodul mit Patientenleitungskabeln, die an einen Patienten zum Sammeln wenigstens eines Vitalzeichens des Patienten anbringbar sind, wobei die Halterung in einer Distanz von der Basis betreibbar ist und die Halterung und die Basis dafür eingerichtet sind, optional miteinander gekoppelt zu werden, um als eine Einheit getragen zu werden, und in zwei getrennte Einheiten trennbar sind; und
einer drahtlosen Kommunikationsverbindung zwischen der Halterung und der Basis, um das wenigstens eine Vitalzeichen von der Halterung zu der Basis zu übertragen, wobei das Defibrillatormodul einen Defibrillationsschock bzw. -stoß auf Grundlage des von der mobilen Einrichtung empfangenen wenigstens einen Vitalzeichens an den Patienten abgibt.

2. Defibrillatorsystem nach Anspruch 1, bei dem die Kommunikationsverbindung durch sowohl eine drahtlose Kommunikationsverbindung als auch eine direkte elektrische Verbindung zwischen der Halterung und der Basis bereitgestellt wird.

3. Defibrillatorsystem nach Anspruch 1, bei dem eine direkte elektrische Verbindung zwischen der Halterung und der Basis als eine alternative Kommunikationsverbindung zu der drahtlosen Kommunikationsverbindung dient.

4. Defibrillatorsystem nach Anspruch 1, bei dem die Kommunikationsverbindung eine direkte elektrische Verbindung zwischen der Basis und zwei oder mehreren der Halterungen beinhaltet.

5. Modulares externes Defibrillatorsystem nach Anspruch 1, bei dem die Basis einen Überwachungs- bzw. Monitorbereich aufweist, um die Vitalzeichen des Patienten visuell anzuzeigen.

6. Defibrillatorsystem nach Anspruch 1, bei dem die durch die Halterung überwachten Vitalzeichen des Patienten über mehrere Leitungen erhaltene EKG-Daten, nichtinvasive Blutdruckdaten, Pulsoximeterdaten, Kapnographiedaten und respiratorische Daten, invasive Blutdruckabfragen und/oder Patiententemperaturdaten beinhalten.

7. Defibrillatorsystem nach Anspruch 5, bei dem der Monitorbereich der Basis über mehrere Leitungen erhaltene EKG-Daten, nichtinvasive Blutdruckdaten, Pulsoximeterdaten, Kapnographiedaten und respiratorische Daten, invasive Blutdruckabfragen und/oder Patiententemperaturdaten anzeigt.

8. System nach Anspruch 1, bei dem das Defibrillatormodul auf Grundlage der Vitalzeichen des Patienten Defibrillationsstöße mit dem intrinsischen Rhythmus des Patienten synchronisiert.

9. System nach Anspruch 1, bei dem die Basis ein Dateninterprätationsmodul beinhaltet, das die Vitalzeichen des Patienten analysiert, um interprätative Aussagen über den kardialen oder respiratorischen Zustand des Patienten zu treffen.

10. System nach Anspruch 1, bei dem die Basis eine Arretieranordnung zum Anbringen der Halterung in einer freigebbaren Weise aufweist, wobei die Halterung in einer Distanz von der Basis betreibbar ist.

11. System nach Anspruch 10, bei dem die Aritierungsanordnung eine Aussparung zur Aufnahme der Halterung aufweist.

12. System nach Anspruch 11, bei dem die Aussparung in freigebbarer Weise eine oder mehrere Halterungen aufnehmen bzw. halten kann.

13. System nach Anspruch 11, bei dem die Aussparung zwei der Halterungen freigebbar anbringt bzw. aufnimmt.

14. System nach Anspruch 13, bei dem die zwei Halterungen aneinander verriegelt bzw. miteinander arretiert sind und über einen Verbinder auf jeder Halterung kommunizieren.

15. System nach Anspruch 14, bei dem die Basis getrennte Verbinder zum Kommunizieren mit jeder Halterung beinhaltet.

16. System nach Anspruch 14, bei dem die Halterungen unterschiedliche Vitalzeichen überwachen.

17. System nach Anspruch 10, bei dem die Basis eine Anzahl von Basis-Interfaceverbindern aufweist, um eine direkte elektrische Verbindung mit einer Anzahl von Halterungen in der Arretieranordnung bereitzustellen.

18. System nach Anspruch 17, bei dem jeder Interfaceverbinder einer Halterung mit einem Interfaceverbinder einer anderen Halterung ausgerichtet und verbunden werden kann.

19. System nach Anspruch 10, bei dem die Arretieranordnung ferner eine Arretierung zum Halten der Halterung in der Aussparung aufweist.

20. System nach Anspruch 19, bei dem die Arretierung mit einem Hohlraum auf der Halterung ausgerichtet ist bzw. wird, um die Halterung in der Aussparung zu halten.

21. Kardiales Therapiesystem nach Anspruch 19, bei dem die Arretierung federbetrieben ist.

22. System nach Anspruch 19, bei dem die Arretieranordnung ferner einen Druckknopf aufweist, der die federbetriebene Arretierung freigibt, wenn ein Benutzer die Halterung aus dem Schlitz bzw. der Aufnahme entfernen bzw. entnehmen will.

23. System nach Anspruch 19, bei dem die Arretieranordnung ferner wenigstens eine Feder aufweist, die in wenigstens einer Führungsrippe bzw. -nut angeordnet ist.

24. System nach Anspruch 19, wobei die Arretieranordnung in Wirkverbindung mit der Basis steht und mit einem passenden Hohlraum auf der Halterung ausgerichtet ist.

25. System nach Anspruch 1, bei dem die Basis eine Aussparung aufweist, in die die Halterung in einer freigebbaren Weise anzubringen ist, wobei die Halterung in einer Distanz von der Basis betreibbar ist.

26. System nach Anspruch 25, bei dem die Aussparung in freigebbarer Weise eine Leistungsversorgung für die Basis halten kann.

27. System nach Anspruch 25, bei dem die Aussparung dafür eingerichtet ist, unterschiedliche Größen von Halterungen anzubringen bzw. aufzunehmen, wobei wenigstens eine Halterung an einer Arretieranordnung befestigt ist.

28. System nach Anspruch 25, bei dem Arretieranordnung ein Paar Führungsrippen in der Aussparung aufweist, um die Halterungen aufzunehmen und die Bewegung der Halterung in sowohl der horizontalen als auch der vertikalen Richtung zu steuern.

29. System nach Anspruch 28, bei dem die Führungsrippen die Halterung während der Einführung in die Aussparung ausrichten, um eine elektrische Verbindung zwischen einem Basis-Interfaceverbinder und einem Interfaceverbinder der Halterung, die zusammen die Kommunikationsverbindung bereitstellen, sicherzustellen.

30. Kardiales Therapiesystem nach Anspruch 29, bei dem die Führungsrippen der Arretieranordnung einen Interfaceverbinder einer Halterung mit einem Interfaceverbinder einer Basis ausrichten, um die direkte elektrische Verbindung einzurichten.

31. System nach Anspruch 25, bei dem die Basis Einfügungen aufweist, um Defibrillationspaddel, einen Tragebeutel und/oder eine Befestigungshalterung, die die Halterung hält, beinhaltet.

32. System nach Anspruch 25, bei dem die Basis Leistung bereitstellt, um eine Batterie, die die Halterung speist, zu laden.

33. System nach Anspruch 32, bei dem die Basis Ladeleistung drahtlos an die Halterung bereitstellt.

34. System nach Anspruch 25, bei dem das kardiale Therapiesystem auf Grundlage der Patientendaten die elektrische kardiale Therapie mit dem intrinsischen Rhythmus des Patienten synchronisiert.

35. System nach Anspruch 1, das ferner aufweist:
eine Dockingstation (8) um die Basis in einer freigebbaren Weise aufzunehmen, wobei die Basis betreibbar ist, wenn sie durch die Dockingstation aufgenommen ist oder sich in einer Distanz von der Dockingstation befindet.

36. Kardiales Therapiesystem nach Anspruch 35, wobei die Dockingstation die Halterung in einer freigebbaren Weise aufnimmt.

37. System nach Anspruch 35, bei dem die Dockingstation Leistung zum Wiederaufladen von Batterien in der Basis und zum Speisen der Basis bereitstellt.

38. System nach Anspruch 35, bei dem die Dockingstation Leistung zum Wiederaufladen einer Batterie in der Halterung bereitstellt.

## Revendications

1. Système de défibrillation externe modulaire, comportant :
une base (14) contenant un module de défibrillation ;
une ensemble portable ou une gaine (12) ayant un module de paramètres de patient comportant des câbles de dérivation de patient pouvant être fixés à un patient pour collecter au moins un signe vital du patient, la gaine étant opérationnel à une certaine distance de la base, la gaine et la base étant adaptés pour être couplés ensemble de manière facultative pour être transportés en tant qu'unité et pouvant être séparés en unités séparées ; et
une liaison de communication sans fil entre la gaine et la base pour acheminer le au moins un signe vital depuis la gaine jusqu'à la base, le module de défibrillation délivrant un choc de défibrillation à un patient sur la base d'au moins un signe vital reçu en provenance de la gaine.

2. Système de défibrillation selon la revendication 1, dans lequel la liaison de communication est fournie à la fois par une liaison de communication sans fil et une connexion électrique directe entre la gaine et la base.

3. Système de défibrillation selon la revendication 1, dans lequel une connexion électrique directe entre la gaine et la base sert de liaison de communication alternative à la liaison de communication sans fil.

4. Système de défibrillation selon la revendication 1, dans lequel la liaison de communication inclut une connexion électrique directe entre la base et deux ou plus des gaines.

5. Système de défibrillation externe modulaire selon la revendication 1, dans lequel la base a une zone de surveillance ou d'écran pour afficher visuellement les signes vitaux du patient.

6. Système de défibrillation selon la revendication 1, dans lequel les signes vitaux du patient surveillés par la gaine incluent une ou plusieurs données parmi des données ECG multi-électrodes, des données de pression sanguine non-invasive, des données de sphygmo-oxymètre, des données de capnographie et des données respiratoires, des lectures de pression sanguine invasive, et des données de température du patient.

7. Système de défibrillation selon la revendication 5, dans lequel la zone de surveillance de la base affiche visuellement une ou plusieurs données parmi des données ECG multi-électrodes, des données de pression sanguine non-invasive, des données de sphygmo-oxymètre, des données de capnographie, des lectures de pression sanguine invasive, et des données de température du patient.

8. Système selon la revendication 1, dans lequel le module de défibrillation synchronise des chocs de défibrillation sur le rythme intrinsèque du patient sur la base des signes vitaux du patient.

9. Système selon la revendication 1, dans lequel la base inclut un module d'interprétation de données qui analyse les signes vitaux du patient pour former des instructions interprétatives sur la condition cardiaque ou respiratoire du patient.

10. Système selon la revendication 1, dans lequel la base a un ensemble de verrouillage pour monter la gaine d'une manière libre, la gaine étant opérationnel à une certaine distance de la base.

11. Système selon la revendication 10, dans lequel l'ensemble de verrouillage a une niche pour recevoir la gaine.

12. Système selon la revendication 11, dans lequel la niche peut contenir de manière libre un ou plusieurs gaines.

13. Système selon la revendication 11, dans lequel la niche monte de manière libre deux des gaines.

14. Système selon la revendication 13, dans lequel les deux gaines sont verrouillés ensemble et communiquent via un connecteur sur chaque gaine.

15. Système selon la revendication 14, dans lequel la base inclut des connecteurs séparés pour communiquer avec chaque gaine.

16. Système selon la revendication 14, dans lequel les gaines surveillent différents signes vitaux.

17. Système selon la revendication 10, dans lequel la base a une pluralité de connecteurs d'interface de base pour fournir une connexion électrique directe avec une pluralité d'gaines dans l'ensemble de verrouillage.

18. Système selon la revendication 17, dans lequel chaque connecteur d'interface de la gaine peut être aligné et connecté à un autre connecteur d'interface de la gaine.

19. Système selon la revendication 10, dans lequel l'ensemble de verrouillage comporte en outre un verrou pour maintenir la gaine dans la niche.

20. Système selon la revendication 19, dans lequel le verrou s'aligne sur une cavité située sur la gaine pour maintenir la gaine dans la niche.

21. Système de thérapie cardiaque selon la revendication 19, dans lequel le verrou est à ressort.

22. Système selon la revendication 19, dans lequel l'ensemble de verrouillage comporte en outre un bouton-poussoir qui libère le verrou à ressort lorsqu'un utilisateur veut retirer la gaine de la fente.

23. Système selon la revendication 19, dans lequel l'ensemble de verrouillage comporte en outre au moins un ressort localisé dans au moins une nervure de guidage.

24. Système selon la revendication 19, dans lequel le verrou est couplé de manière opérationnelle à la base et s'aligne sur une cavité d'ajustement située sur la gaine.

25. Système selon la revendication 1, dans lequel la base a une niche dans laquelle la gaine est monté d'une manière libre, la gaine étant opérationnel à une certaine distance de la base.

26. Système selon la revendication 25, dans lequel la niche peut contenir de manière libre un bloc d'alimentation pour la base.

27. Système selon la revendication 25, dans lequel la niche est adaptée pour monter différentes tailles d'gaines, au moins une gaine étant fixé à un ensemble de verrouillage.

28. Système selon la revendication 25, dans lequel l'ensemble de verrouillage a une paire de nervures de guidage dans la niche pour recevoir la gaine et commander le déplacement de la gaine à la fois dans la direction horizontale et dans la direction verticale.

29. Système selon la revendication 28, dans lequel les nervures de guidage alignent la gaine pendant l'insertion dans la niche pour garantir une connexion électrique entre un connecteur d'interface de la base et un connecteur d'interface de la gaine qui fournissent ensemble la liaison de communication.

30. Système de thérapie cardiaque selon la revendication 29, dans lequel les nervures de guidage de l'ensemble de verrouillage alignent un connecteur d'interface de la gaine sur un connecteur d'interface de la base pour établir la connexion électrique directe.

31. Système selon la revendication 25, dans lequel la base inclut des tampons pour fixer au moins l'un parmi des palettes de défibrillation, un sac de transport, et un support de montage de gaine qui contient la gaine.

32. Système selon la revendication 25, dans lequel la base délivre du courant pour charger une batterie qui alimente la gaine.

33. Système selon la revendication 32, dans lequel la base délivre un courant de charge à la gaine d'une manière sans fil.

34. Système selon la revendication 25, dans lequel le module de thérapie cardiaque synchronise la thérapie cardiaque électrique sur le rythme intrinsèque du patient sur la base des données du patient.

35. Système selon la revendication 1, comportant en outre :
une station d'accueil (8) pour héberger la base d'une manière libre, la base étant opérationnelle lorsque hébergée par la station d'accueil ou à une certaine distance par rapport à la station d'accueil.

36. Système de thérapie cardiaque selon la revendication 35, dans lequel la station d'accueil héberge la gaine d'une manière libre.

37. Système selon la revendication 35, dans lequel la station d'accueil délivre du courant pour recharger des batteries dans la base et alimenter la base.

38. Système selon la revendication 35, dans lequel la station d'accueil délivre du courant pour recharger une batterie dans la gaine.
